# EUROPEAN PATENT SPECIFICATION

(11) **EP 1 124 603 B1**
(45) Date of publication and mention of the grant of the patent: **26.11.2003**
(21) Application number: 99952193.3
(22) Date of filing: 27.10.1999
(51) Int. Cl.: A61M 11/06, B05B 11/00

(54) **VAPORIZING DEVICE FOR ADMINISTERING STERILE MEDICATION**
VERDAMPFUNGSVORRICHTUNG ZUR ABGABE VON STERILEN MEDIKAMENTEN
DISPOSITIF DE VAPORISATION POUR ADMINISTRER DES MEDICAMENTS STERILES

(30) Priority: 27.10.1998 US 105726 P; 16.02.1999 US 250272
(43) Date of publication of application: 22.08.2001
(73) Proprietor: WEBB, Garth T., White Rock, British Columbia V4P 1M6 (CA)
(72) Inventor: WEBB, Garth T., White Rock, British Columbia V4P 1M6 (CA)
(74) Representative: Eisenführ, Speiser & Partner
(86) International application number: CA9901002
(87) International publication number: WO00024444

(56) References cited:
- EP-A- 0 562 943
- WO-A-96/03344
- WO-A-97/18902
- WO-A-97/29799
- DE-A- 4 403 755
- US-A- 5 492 252
- US-A- 5 620 434

## Description

### Technical Field

The invention relates to the field of devices for administering medicated liquids, and more particularly to vaporizers.

### Background Art

It is common to use vaporizers or atomizers for delivering certain medicines to humans, such as asthma medication. Many medications can be effectively delivered into the bloodstream by inhalation through the lungs. However, many medications, such as insulin, must be delivered in sterile form, which precludes the use of existing vaporizers, in which the medication passes through unsterilized passages, including the vaporizer itself. There is therefore a need for a vaporizer which can administer sterile medications.

EP-A-0 562 943 discloses a double-chambered package for the storing, then mixing of two components. It uses two necks at either end of the container, one end forming a mouth with a pump and the other end carrying a capsule which can be broken by a cutting element to release one of the components into the other. WO 97 18902 A (URSATEC VERPACKUNG GMBH) discloses a fluid dispensing apparatus in which filters for degerminating air are provided in the air intake area and the fluid outlet. DE 44 03 775 A (PFEIFFER ERICH GMBH & CO KG) also discloses a double-chambered package for the storing of two components.

### Disclosure of Invention

The invention therefore provides a device for dispensing a vaporized spray of sterile medicated liquid, comprising a housing for containing a supply of sterile medicated liquid, means for pressurizing the liquid, and a combination atomizing and pressure release valve.

The invention also provides a combination atomizing and pressure release valve comprising a layer of flexible material provided with an aperture, and a plug extending upwardly through the aperture and having a surface against which the layer of flexible material is biassed in the closed position, the layer lifting off the surface of the plug in the open position to thereby open the aperture.

### Brief Description of Drawings

In drawings which illustrate a preferred embodiment of the invention:

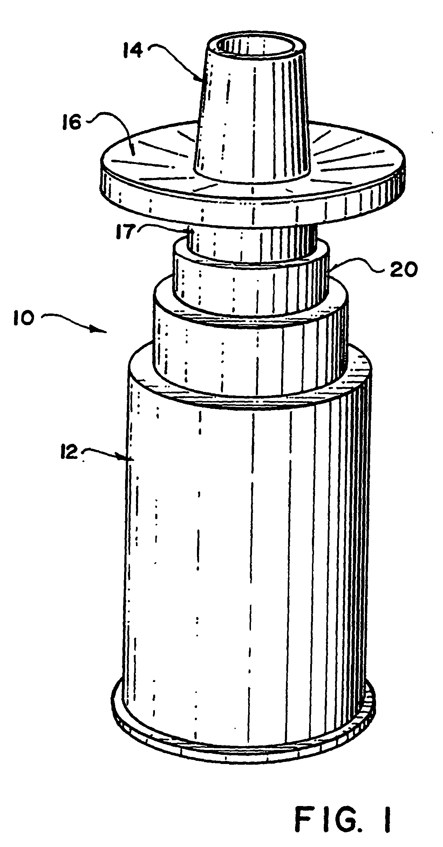

Fig. 1 is a perspective view of the invention;
Fig. 2 is a vertical cross-section of the invention as shown in Fig. 1;
Fig. 3 is a detail of the atomizer portion of the invention;
Fig. 4 is a top view taken along lines 4-4 of Fig. 3;
Fig. 5 is a vertical cross-section of a second embodiment of the invention;
Fig. 6 is a vertical cross-section of a third embodiment of the invention;
Fig. 7 is a partial elevation of the mouthpiece according to one embodiment of the invention; and
Fig. 8 is an end view of the mouthpiece shown in Fig. 8.

### Best mode(s) For Carrying Out the Invention

The vaporizer 10 of the invention has a hollow body or housing 12, such as of molded plastic, and a tubular mouthpiece 14 connected to a circular disc 16 which receives the user's lips. The lower end of mouthpiece 14 forms a cylindrical piston 17 which slides in chamber 18 formed by cylindrical walls 20. A slight gap between piston 17 and the inner surface of walls 20 permits the passage of air. Extending downwardly and co-axially from piston 17 is an inner piston 22 which sealingly slides within chamber 24 formed by walls 26. The lower end of piston 22 is sealed at seal 28. Piston 22 has a central hollow tube 30 which bears against spring 32 within cylindrical chamber 36. Spring 32 in turn bears against ball valve 34 seated in opening 40 which communicates with passage 42 extending downwardly through inlet tube 38 which in turn communicates with the hollow interior 44 of housing 12. A flexible, expandable bag 50 is sealingly secured at 52 to tube 38 and at 54 to walls 26. Air passage 56 permits air to pass from chamber 18 into the interior of flexible bag 50, which therefore expands as the liquid in chamber 44 is dispensed.

A soluble medication tablet or powder 60 is provided in the lower end of housing 12. A layer of breakable aluminum foil 62 or similar material is provided across circular opening 64 at the lower end of housing 12. A cylindrical piston 66 slides with a friction fit within chamber 68 formed by circular wall 70. The upper surface 72 of cylinder 66 bears against tablet 60. Rim 74 limits the upward travel of cylinder 66.

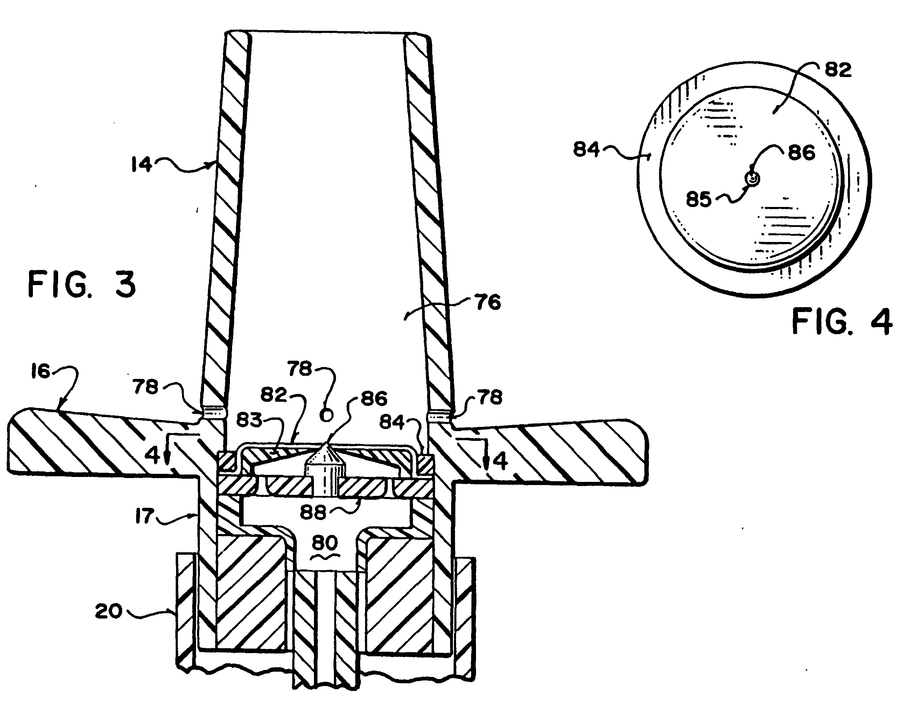

The vaporizer section of the invention is shown in detail in Fig. 3. Chamber 80 communicates with tube 30. Pressure release valve/atomizer 82 is formed of a flexible material such as rubber. It extends across chamber 80 and has a central hole 85, and is supported and secured in place at its ends by valve support 83 and ring 84. A conical plug 86 is supported on the central axis of the housing by support 88. The upper end of plug 86 extends through the hole 85 in a manner that pressure is placed on pressure release valve 82 so the edges of hole 85 seal against the surface of plug 86 in the rest position, but when pressure is applied to the lower surface of valve 82 it lifts off the surface of plug 86. The surface of plug 86 and/or the lower surface of rubber valve 82 may be provided with swirl patterns to facilitate vaporization. Air holes 78 are provided in mouthpiece 14 to provide air circulation to assist in vaporization.

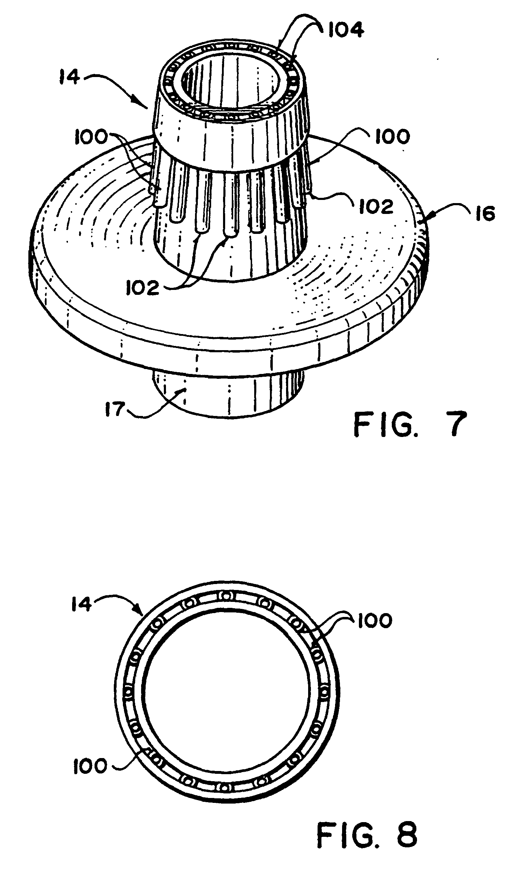

While plug 86 is shown as conical in shape with a pointed vertex, it could also have a blunt end, or be in the shape of a sphere, hemisphere or paraboloid, or similar smoothly curved surface. In that case, where the upper contact surface of the plug 86 is spherical or rounded, vaporization could be achieved by providing an array or multiplicity of small holes in the valve 82 in the area of contact of the plug 86 with the valve 82, rather than a single larger hole as shown. Other forms of venting of the mouthpiece to assist vaporization are also possible. For example, as shown in Fig. 7, a circular array of straw-like tubes 100 is provided extending along the outside of the mouthpiece 14 parallel to the axis of the device, open to the atmosphere at their lower ends 102. The upper ends 104 of tubes 100 open into the mouth of the user. A cylinder 106 encircles the upper portion of tubes 100 in the area where the user's lips will encircle the mouthpiece 14, and may fill in the spaces between the tubes 100. In this way passage of the vaporized medicine to user's lungs is facilitated, since the array of tubes forms a cylindrical sheath of air around the central spray of medicine. Various patterns for the tubes 100 can be provided, such as a spiral around mouthpiece 14. The upper ends 104 of tubes 100 can also be deflected to create an airfoil effect to speed the passage of the spray of medicine.

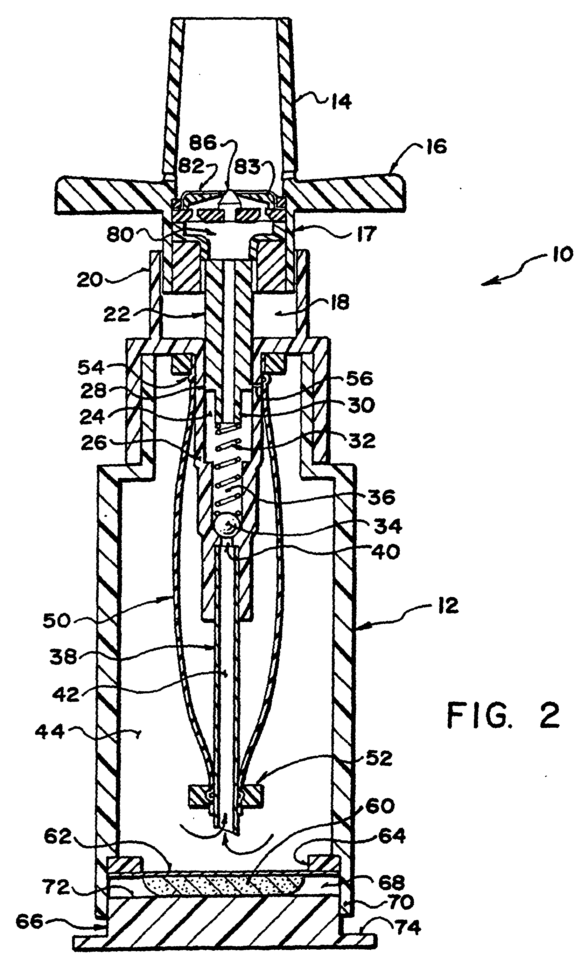

In operation, chamber 44 is filled with a sterile liquid, such as a sterile saline solution, when the device is manufactured. The device is not designed to be refilled, and so is disposed after the liquid has been used up. When the user wishes to commence use of the device, piston 66 is pressed into the housing 12, causing tablet 60 to be forced through aluminum foil layer 62 into the sterile liquid in chamber 44, where it dissolves. The friction fit of piston 66 against walls 70 prevents leakage. To dispense the vaporized medicated liquid through mouthpiece 14, disc 16 is drawn up to the position shown in Fig. 2, which unseats ball valve 34 and draws the sterile medicated liquid up through tubes 38 and 30 into chambers 24 and 80. Disc 16 is then forced downwardly against the resistance of spring 32, causing pressure release valve 82 to lift off plug 86, allowing the liquid to spray through hole 85, and be vaporized due to the interaction of the hole 85 and plug 86. Atmospheric air is able to penetrate into chamber 18, and then through hole 56 into the interior of flexible bag 50, which expands to replace the volume of liquid which is expelled.

Preferably plug 86 is plated with silver to provide a biostatic barrier by creating silver ions which repel bacteria from entering hole 85. Similarly a small silver pellet could be added to the sterile liquid in chamber 44 to act as a mild preservative in maintaining the sterility of the liquid. While the distance between the atomizing valve 82 and the pump arrangement 22/28 is shown as quite short in Fig. 2, this obviously could be made much longer by interposing a length of flexible conduit or the like.

Other constructions can be used to achieve the same effect as flexible bag 50. For example, the sterile liquid can be contained within a flexible bag within chamber 44. Tube 38 would then end within the liquid bag. There would be no air access 56 to the bag, but atmospheric air could enter around the bag in chamber 44 through an air passage in housing 12. In that event the medicine tablet 60 would be contained within the flexible liquid bag, arranged so that it could be dispensed into the liquid by pressing it through a foil seal. Another alternative to bag 50 is to load the sterile liquid in chamber 44 with a pressurized gas, as in an aerosol spray, so that depression of the mouthpiece 14/16 causes the pressurized gas and liquid to be released under pressure into chamber 80.

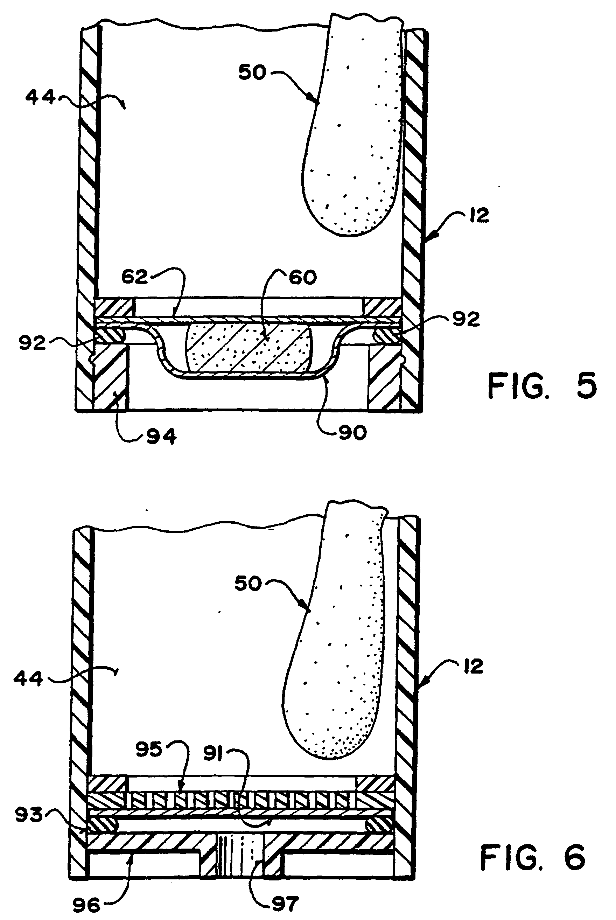

According to the embodiment shown in Figure 5, the air-filled bag 50 is not secured around tube 38 but is loose within housing 12. The interior of bag 50 communicates to the atmosphere exterior to housing 12 through an opening in housing 12. In this embodiment the medicinal tablet 60 is held in place beneath foil layer 62 by a flexible membrane 90 which seals against foil layer 62 by a rubber gasket 92 held in place by retaining ring 94. Tablet 60 is introduced into the sterile liquid in chamber 44 by pressing on membrane 90, thereby forcing tablet 60 through foil layer 62.

In the embodiment shown in Fig. 6, liquid medicine is injected into the chamber 44 through a sterilizing micro-filter 91, rather than dissolving a medicated tablet in the sterile solution. Micro-filter 91 is supported between perforated filter support 95 and rubber gasket 93, which is held in place by support disc 96. Disc 96 has an attachment port into which the end of a syringe sealingly fits. To introduce the sterile medicine into the sterile solution in chamber 44, the syringe is filled with the desired amount of medicinal liquid and the end of the syringe is sealingly inserted into attachment port 97. The plunger of the syringe is depressed until the desired amount of liquid medicine is forced through micro-filter 91, at which point the syringe is removed and a plug (not shown) is then sealingly inserted into port 97.

## Claims

1. A pressure release valve adapted to be used as an atomizer, said pressure release valve comprising a layer of flexible material (82) having an upper surface and a lower surface and provided with an aperture (85), and a plug (86) extending upwardly through said aperture and having a surface against which said layer of flexible material (82) is biassed in a closed position thereby biassing the edge of said aperture (85) against the surface of said plug (86), said edge of said aperture (85) lifting off the surface of said plug (86) to an open position when sufficient pressure is applied to said lower surface of said layer of flexible material (82) to thereby open the aperture (85), whereby when a liquid under pressure contacts said lower surface of said layer of flexible material (82) and moves said layer of flexible material (82) to said open position, liquid flowing between said edge of said aperture (85) and said surface of said plug (86) is atomized.

2. The pressure release valve of claim 1 wherein said flexible layer (82) is secured at an outer edge thereof and said aperture (85) is centrally located in said flexible layer (82).

3. The pressure release valve of claim 1 wherein said plug (86) has a smoothly curved surface.

4. The pressure release valve of claim 1 wherein said plug (86) is conical.

5. The pressure release valve of claim 1 wherein said flexible layer (82) is provided with a lower surface patterned with raised swirls.

6. The pressure release valve of claim 1 wherein said plug (86) is silver plated.

7. The pressure release valve of claim 1 wherein said flexible material (82) is rubber.

8. A device (10) for dispensing a vaporized spray of sterile medicated liquid, comprising a housing (12) for containing a supply of said sterile medicated liquid, means for pressurizing said liquid (22), and a pressure release valve according to claim 1, said pressure release valve communicating with the interior of said housing (12).

9. The device for dispensing a vaporized spray of sterile medicated liquid according to claim 8 wherein said housing (12) comprises an expandable, flexible bag (50) communicating with the exterior atmosphere.

10. The device for dispensing a vaporized spray of sterile medicated liquid according to claim 8 wherein said liquid is medicated by adding a soluble tablet (60) to said liquid.

11. The device for dispensing a vaporized spray of sterile medicated liquid according to claim 10 wherein said soluble tablet is added to said liquid by pressing said tablet through a breakable sealed barrier (62).

12. The device for dispensing a vaporized spray of sterile medicated liquid according to claim 8 further comprising a mouthpiece (14) having a central aperture communicating with said aperture (85) of said pressure release valve.

13. The device for dispensing a vaporized spray of sterile medicated liquid according to claim 12 wherein said means for pressurizing said liquid comprises pump means connected to said mouthpiece (14) and reciprocable in said housing (12).

14. The device for dispensing a vaporized spray of sterile medicated liquid according to claim 11 wherein said means for adding a sterile medication to said liquid comprises a soluble sterile tablet (60) which is added to said liquid by pressing said tablet through a breakable sealed barrier (62) in said housing (12).

15. The device for dispensing a vaporized spray of sterile medicated liquid according to claim 11 wherein said means for adding a sterile medication to said liquid comprises a soluble sterile powder (60) which is added to said liquid by pressing said powder through a breakable sealed barrier (62) in said housing (12).

16. The device for dispensing a vaporized spray of sterile medicated liquid according to claim 14 wherein said tablet (60) is pressed through said sealed barrier by means of a piston (66) sealingly slidable within said housing (12) and adapted to bear against said tablet (60).

17. The device for dispensing a vaporized spray of sterile medicated liquid according to claim 11 wherein said means for adding a sterile medication to said liquid comprises means for forcing a liquid medicament through a microfilter (91) in said housing (12).

18. The device for dispensing a vaporized spray of sterile medicated liquid according to claim 17 wherein said means for forcing a liquid medicament through a microfilter (91) in said housing comprises a syringe, and said housing (12) comprises means (97) for sealingly receiving an orifice of said syringe in said housing (12).

## Patentansprüche

1. Druckauslösungs-Ventil, ausgebildet zur Benutzung als ein Zerstäuber, wobei das Druckauslösungs-Ventil folgendes umfasst:
eine Schicht aus elastischem Material (82), welche eine obere Oberfläche und eine untere Oberfläche aufweist und mit einer Öffnung (85) ausgestattet ist, und einen Verschluss (86), der sich nach oben hin durch die Öffnung hindurch ausdehnt und eine Oberfläche aufweist, gegen welche die Schicht aus elastischem Material (82) in einer geschlossenen Position vorgespannt ist und dadurch den Rand der Öffnung (85) gegen die Oberfläche des Verschlusses (86) vorspannt, wobei der Rand der Öffnung (85) die Oberfläche des Verschlusses (86) zu einer geöffneten Position anhebt, wenn auf die untere Oberfläche der Schicht aus elastischem Material (82) ausreichender Druck ausgeübt wird, um dadurch die Öffnung (85) zu öffnen, wobei, wenn eine Flüssigkeit unter Druck mit der unteren Oberfläche der Schicht aus elastischem Material (82) in Berührung kommt und die Schicht aus elastischem Material (82) zu der geöffneten Position bewegt, die Flüssigkeit zerstäubt wird, welche zwischen dem Rand der Öffnung (85) und der Oberfläche des Verschlusses (86) hindurchfließt.

2. Druckauslösungs-Ventil nach Anspruch 1, wobei die elastische Schicht (82) an einem äußeren Rand von dieser befestigt ist, und sich die Öffnung (85) in der Mitte der elastischen Schicht (82) befindet.

3. Druckauslösungs-Ventil nach Anspruch 1, wobei der Verschluss (86) eine sanft gebogene Oberfläche aufweist.

4. Druckauslösungs-Ventil nach Anspruch 1, wobei der Verschluss (86) eine kegelförmige Gestalt besitzt.

5. Druckauslösungs-Ventil nach Anspruch 1, wobei die elastische Schicht (82) mit einer Unterseite ausgestattet ist, die mit erhöhten Wirbeln geformt ist.

6. Druckauslösungs-Ventil nach Anspruch 1, wobei der Verschluss (86) versilbert ist.

7. Druckauslösungs-Ventil nach Anspruch 1, wobei das elastische Material (82) Gummi ist.

8. Vorrichtung (10) zur Abgabe eines verdampften Sprays von steriler medizinischer Flüssigkeit, welche ein Gehäuse (12) umfasst, um einen Vorrat von steriler medizinischer Flüssigkeit zu enthalten, ein Mittel zum unter Druck setzen der Flüssigkeit (22) und ein Druckauslösungs-Ventil nach Anspruch 1, wobei das Druckauslösungs-Ventil mit dem Innenraum des Gehäuses (12) in Verbindung steht.

9. Vorrichtung zur Abgabe eines verdampften Sprays von steriler medizinischer Flüssigkeit nach Anspruch 8, wobei das Gehäuse (12) einen dehnbaren, elastischen Beutel (50) umfasst, der mit der äußeren Umgebungsluft in Verbindung steht.

10. Vorrichtung zur Abgabe eines verdampften Sprays von steriler medizinischer Flüssigkeit nach Anspruch 8, wobei, durch das Hinzufügen einer löslichen Tablette (60) zu der Flüssigkeit, eine medizinische Flüssigkeit erzeugt wird.

11. Vorrichtung zur Abgabe eines verdampften Sprays von steriler medizinischer Flüssigkeit nach Anspruch 10, wobei die lösliche Tablette, durch das Hindurchpressen der Tablette durch eine zerbrechliche verschlossene Schicht (62), zu der Flüssigkeit hinzugefügt wird.

12. Vorrichtung zur Abgabe eines verdampften Sprays von steriler medizinischer Flüssigkeit nach Anspruch 8, welche ferner ein Mundstück (14) umfasst, das eine zentrale Öffnung aufweist, die mit der Öffnung (85) des Druckauslösungs-Ventils in Verbindung steht.

13. Vorrichtung zur Abgabe eines verdampften Sprays von steriler medizinischer Flüssigkeit nach Anspruch 12, wobei das Mittel zum unter Druck setzen der Flüssigkeit ein Pumpmittel umfasst, das mit dem Mundstück (14) verbunden ist, und in einem austauschbaren Zustand in dem Gehäuse (12) angeordnet ist.

14. Vorrichtung zur Abgabe eines verdampften Sprays von steriler medizinischer Flüssigkeit nach Anspruch 11, wobei das Mittel zum Hinzufügen eines sterilen Medikaments zu der Flüssigkeit eine lösbare sterile Tablette (60) umfasst, welche durch das Hindurchpressen der Tablette durch eine zerbrechliche verschlossene Schicht (62) in dem Gehäuse (12) zu der Flüssigkeit hinzugefügt wird.

15. Vorrichtung zur Abgabe eines verdampften Sprays von steriler medizinischer Flüssigkeit nach Anspruch 11, wobei das Mittel zum Hinzufügen eines sterilen Medikaments zu der Flüssigkeit ein lösbares steriles Pulver (60) umfasst, welches durch das Hindurchpressen des Pulvers durch eine zerbrechliche verschlossene Schicht (62) in dem Gehäuse (12) zu der Flüssigkeit hinzugefügt wird.

16. Vorrichtung zur Abgabe eines verdampften Sprays von steriler medizinischer Flüssigkeit nach Anspruch 14, wobei die Tablette (60) mittels eines Kolbens (66) durch die verschlossene Schicht gedrückt wird, wobei der Kolben (66) innerhalb des Gehäuses (12) verschließend gleitet und ausgebildet ist, um gegen die Tablette (60) zu drücken.

17. Vorrichtung zur Abgabe eines verdampften Sprays von steriler medizinischer Flüssigkeit nach Anspruch 11, wobei das Mittel zum Hinzufügen eines sterilen Medikaments zu der Flüssigkeit ein Mittel umfasst, zum Hindurchtreiben eines flüssigen Medikaments durch einen Mikrofilter (91 ) in dem Gehäuse (12).

18. Vorrichtung zur Abgabe eines verdampften Sprays von steriler medizinischer Flüssigkeit nach Anspruch 17, wobei das Mittel zum Hindurchtreiben eines flüssigen Medikaments durch einen Mikrofilter (91) in dem Gehäuse eine Spritze umfasst, und das Gehäuse (12) ein Mittel (97) umfasst, zum verschiebenden Empfangen einer Öffnung der Spritze in dem Gehäuse (12).

## Revendications

1. Soupape régulatrice de pression adaptée pour être utilisée en tant qu'atomiseur, ladite soupape régulatrice de pression comprenant une couche de matériau flexible (82) ayant une surface supérieure et une surface inférieure et étant munie d'une ouverture (85), et d'un obturateur (86) s'étendant vers le haut à travers ladite ouverture et ayant une surface contre laquelle ladite couche de matériau flexible (82) est sollicitée dans une position fermée, sollicitant par là même l'arête de ladite ouverture (85) contre la surface dudit obturateur (86), ladite arête de ladite ouverture (85) quittant la surface dudit obturateur (86) vers une position ouverte lorsqu'une pression suffisante est appliquée sur ladite surface inférieure de ladite couche de matériau flexible (82) pour ouvrir ainsi l'ouverture (85), moyennant quoi lorsqu'un liquide sous pression vient en contact avec ladite surface inférieure de ladite couche de matériau flexible (82) et déplace ladite couche de matériau flexible (82) dans ladite position ouverte, du liquide s'écoulant entre ladite arête de ladite ouverture (85) et ladite surface dudit obturateur (86) est atomisé.

2. Soupape régulatrice de pression selon la revendication 1, dans laquelle ladite couche flexible (82) est fixée à une arête extérieure de celle-ci et dans laquelle ladite ouverture (85) est située au centre de ladite couche flexible (82).

3. Soupape régulatrice de pression selon la revendication 1, dans laquelle ledit obturateur (86) possède une surface légèrement incurvée.

4. Soupape régulatrice de pression selon la revendication 1, dans laquelle ledit obturateur (86) est conique.

5. Soupape régulatrice de pression selon la revendication 1, dans laquelle ladite couche flexible (82) est munie d'une surface inférieure structurée en ondulations surélevées.

6. Soupape régulatrice de pression selon la revendication 1, dans laquelle ledit obturateur (86) est plaqué à l'argent.

7. Soupape régulatrice de pression selon la revendication 1, dans laquelle ledit matériau flexible (82) est du caoutchouc.

8. Dispositif (10) pour délivrer un spray vaporisé de liquide médicamenteux stérile, comprenant un logement (12) destiné à contenir une réserve dudit liquide médicamenteux stérile, des moyens pour mettre ledit liquide (22) sous pression, et une soupape régulatrice de pression selon la revendication 1, ladite soupape régulatrice de pression communiquant avec l'intérieur dudit logement (12).

9. Dispositif pour délivrer un spray vaporisé de liquide médicamenteux stérile selon la revendication 8, dans lequel ledit logement (12) comprend un sac flexible, extensible (50) communiquant avec l'atmosphère extérieure.

10. Dispositif pour délivrer un spray vaporisé de liquide médicamenteux stérile selon la revendication 8, dans lequel ledit liquide est rendu médicamenteux en ajoutant un comprimé soluble (60) audit liquide.

11. Dispositif pour délivrer un spray vaporisé de liquide médicamenteux stérile selon la revendication 10, dans lequel ledit comprimé soluble est ajouté audit liquide en comprimant ledit comprimé à travers une barrière scellée cassable (62).

12. Dispositif pour délivrer un spray vaporisé de liquide médicamenteux stérile selon la revendication 8, comprenant en outre un embout buccal (14) ayant une ouverture centrale communiquant avec ladite ouverture (85) de ladite soupape régulatrice de pression.

13. Dispositif pour délivrer un spray vaporisé de liquide médicamenteux stérile selon la revendication 12, dans lequel lesdits moyens pour mettre ledit liquide sous pression comprennent des moyens formant pompe reliés audit embout buccal (14) et rétractables dans ledit logement (12).

14. Dispositif pour délivrer un spray vaporisé de liquide médicamenteux stérile selon la revendication 11, dans lequel lesdits moyens pour ajouter une médication stérile audit liquide comprennent un comprimé stérile soluble (60) qui est ajouté audit liquide en comprimant ledit comprimé à travers une barrière scellée cassable (62) dans ledit logement (12) .

15. Dispositif pour délivrer un spray vaporisé de liquide médicamenteux stérile selon la revendication 11, dans lequel lesdits moyens pour ajouter une médication stérile audit liquide comprennent une poudre stérile soluble (60) qui est ajoutée audit liquide en comprimant ladite poudre à travers une barrière scellée cassable (62) dans ledit logement (12).

16. Dispositif pour délivrer un spray vaporisé de liquide médicamenteux stérile selon la revendication 14, dans lequel ledit comprimé (60) est comprimé à travers ladite barrière scellée au moyen d'un piston (66) pouvant coulisser de manière hermétique à l'intérieur dudit logement (12) et adapté pour venir s'appuyer contre ledit comprimé (60).

17. Dispositif pour délivrer un spray vaporisé de liquide médicamenteux stérile selon la revendication 11, dans lequel lesdits moyens pour ajouter une médication stérile audit liquide comprennent des moyens pour contraindre un médicament liquide à traverser un microfiltre (91) dans ledit logement (12).

18. Dispositif pour délivrer un spray vaporisé de liquide médicamenteux stérile selon la revendication 17, dans lequel lesdits moyens pour contraindre un médicament liquide à traverser un microfiltre (91) dans ledit logement comprennent une seringue, et ledit logement (12) comprend des moyens (97) pour recevoir de manière hermétique un orifice de ladite seringue dans ledit logement (12).
